# EUROPEAN PATENT APPLICATION

(11) **EP 3 370 175 A1**
(43) Date of publication of application: **05.09.2018**
(21) Application number: 17159181.1
(22) Date of filing: 03.03.2017
(51) Int. Cl.: G06F 19/00, A61B 5/00

(54) **METHOD FOR PROVIDING A WEARABLE DEVICE, METHOD FOR PREDICTING AN ACUTE EXACERBATION AND SYSTEM FOR PREDICTING AN ACUTE EXACERBATION**

(71) Applicant: Linde Aktiengesellschaft, 80331 München (DE)
(72) Inventor: The designation of the inventor has not yet been filed

(57) **Abstract**

The present invention pertains to a method for selecting a wearable device for remotely monitoring a patient with a chronic disease, in particular a COPD patient, the method comprising the steps of:
- specifying a plurality of patient groups (A₁,...,A_{N}), wherein each of the patient groups (A₁,...A_{N}) is defined by at least a set of status parameters of the patient's current health status and/or the patient's clinical history data;
- assigning each of the patient groups (A₁,...,A_{N}) to a respective set of monitoring parameters (P_{N,1},...,P_{N,L});
- for each of the patient groups (A₁,...,A_{N}), selecting at least one wearable device (14) that includes measurement sensors for remotely measuring at least one of the monitoring parameters (P_{N,1},..., P_{N,L}) of the respective set of monitoring parameters (P_{N,1},...,P_{N,L}) of the patient group (A₁,...,A_{N}).

The invention also includes a method and a system for predicting an acute exacerbation of a health condition of a patient with a chronic disease using the selected wearable device for remote measuring of the monitoring parameters, in order to analyse the measured parameter values and to produce a warning signal if a probability of acute exacerbation exceeds a predetermined value.

## Description

### Technical Field

The invention relates to a method for providing a wearable device for remotely monitoring a patient with a chronic disease, a method for predicting an acute exacerbation of a health condition of a patient and a system for predicting an acute exacerbation.

### Technological Background

With changes in life expectancy, diet and activity (particularly in industrialized countries), the number of people with at least one chronic disease is growing rapidly since the prevalence of chronic degenerative disease increases with age. This not only increasingly shifts the major cause of death to chronic diseases, but also provides a major economic burden for society. Chronic diseases include so-called *"diseases of affluence",* as, for example, (chronic) cardiovascular diseases, stroke, cancer, chronic respiratory diseases and diabetes.

As an example, chronic obstructive pulmonary disease (COPD) is one of the most common chronic respiratory diseases worldwide with a continuous increase seen in incidence and prevalence. Currently, more than 64 million estimated people globally (WHO Fact sheet No 315, 2011) suffer from COPD and it is a major cause of mortality, resulting in 3 million deaths per year (WHO 2015) one of the leading causes of death. According to the World Health Organization (WHO), COPD will soon be the third-leading cause of death worldwide.

COPD is a complex disease, generally divided into four categories using the Global Initiative for Chronic Obstructive Lung Disease (GOLD) classification system. It can also co-exist with one or several comorbidities such as, for example, cardiovascular diseases, diabetes, rheumatic diseases, asthma or depression.

Another example of an increasingly common severe chronic disease is congestive heart failure (CHF). CHF is also one of the leading diseases, with more than 20 Million estimated cases worldwide. In developed countries, around 2% of adults have CHF, wherein the rate increases to 6-10% for those over the age of 65. In the year after diagnosis, the risk of death is about 35%, which makes the death rate of patients that suffer from CHF comparable to those with cancer. CHF is classified into four functional classes according to the New York Heart Association and/or into four stages of heart failure according to the American College of Cardiology/American Heart Association. As with COPD, CHF can co-exist with one or several comorbidities.

In general, chronic diseases may be characterized by episodic worsenings of the patient's condition called acute exacerbations. These exacerbations may lead to hospitalization of the patient, represent a major component of the aggregate costs of care and are the main cause for increasing morbidity, morbidity and loss of quality of life of the patients (and their relatives). As the disease advances, the acute exacerbations become more frequent. For some conditions such as COPD, these acute worsenings may also lead to acceleration of the deterioration of the underlying condition.

As an example, a study showed that out of 1404 patients with a diagnosis of an acute exacerbation of CHF, 539 (corresponding to 38%) patients died within one year of their index hospitalization (J. W. Levenson, E. P. McCarthy, J. Lynn, R. B. Davis, R. S. Phillips; "The last six months of life for patients with congestive heart failure"; J. Am. Geriatr. Soc., 2000; 48 (5 Suppl):S101-9).

For COPD, a study showed that out of 430 patients that had to be treated at hospital because of an acute exacerbation of COPD (AECOPD), 63% needed a further at hospital treatment within 1,1 years and 29% died within this time period (J. Garcia-Aymerich, E. Farrero, M. A. Felez, et al.; "Risk factors of readmission to hospital for a COPD exacerbation: a prospective study." Thorax 2003; 58: 100-105).

Furthermore, the recurrence rate is high and twenty per cent of COPD patients are readmitted for acute exacerbation (AECOPD) within 30 days of discharge (Crisafulli E, Torres A, Huerta A, et al. C-Reactive Protein at Discharge, Diabetes Mellitus and ≥ 1 Hospitalization During Previous Year Predict Early Readmission in Patients with Acute Exacerbation of Chronic Obstructive Pulmonary Disease. COPD 2015; 12: 306-14).

In view of these high morbidity and recurrence rates, constant monitoring of patients that have suffered an acute exacerbation is advisable in order to avoid further worsening of the patient's condition. However, monitoring at hospital not only results in loss of quality in life of the patients and thus in psychological comorbidities but is also expensive. A mechanism to regularly measure parameters and predict an acute exacerbation before even the onset of clinical symptoms consistent with an exacerbation might prevent or reduce the length of such a hospitalization.

In particular for COPD, the usefulness of a number of potential predictors for an acute exacerbation (AECOPD) have been investigated in the past and discussed in a large number of scientific publications. Remote monitoring of certain physiological parameters in the patient's home and their analysis using algorithms have also been investigated widely in the past. However, conflicting results in terms of proof of net benefit, quantum of benefit, high variability in patient populations and measured parameters/interventions mean that the evidence of benefit remains unclear. Furthermore, due to the highly complex and individual nature of disease progression in any given patient, the selection of the parameters to be monitored and the sensors to measure them is a highly complex task with considerable uncertainty and intrinsic inter patient variability.

Documents US 2016/029971 A1 and US 2011/201901 A1 both describe a system and a method for predicting a worsening of a patient's disease state.

### Summary of the invention

In view of the technological background laid out above, it is one objective of the present invention to provide a method for providing a wearable device for remotely monitoring a patient with a chronic disease, in particular COPD. Further objectives of the present invention are to provide a method and a system for predicting an acute deterioration in the health of the patient with a chronic disease, in particular of a COPD patient.

These objectives are achieved by the methods and the system according to the independent claims. Preferred embodiments are given in the dependent claims, the description, and the embodiments and examples explained in connection with the figures.

Accordingly, a method for providing a wearable device for remotely monitoring a patient with a chronic disease, in particular COPD, is provided. The method comprises a step of specifying a plurality of patient groups, wherein each of the patient groups is defined by at least a set of status parameters of the patient's current health status and/or the patent's clinical history data. The method further comprises the step of assigning each of the patient groups to a respective set of monitoring parameters and, for each of the patient groups, selecting at least one wearable device that includes measurement sensors for remotely measuring at least one of the monitoring parameters of the respective set of monitoring parameters of the patient group. It is possible for the steps to be performed in the order specified above or in a different order.

One key idea of the method for providing a wearable device is to specify a plurality of patient groups, each of the patient groups being assigned to an individual set of monitoring parameters. By this, the wearable device used for remote monitoring can be adapted to the specific monitoring needs of each of the patient groups. Furthermore, the allocation of the wearable device to a particular patient can be carried out in the most cost efficient manner.

In contrast to a wearable device that may only measure a fixed set of monitoring parameters, the method according to the present invention takes into account the current health status of the patient when assigning the monitoring parameters that have to be measured and thus allows for selection of a wearable device that is adapted for remotely measuring only the monitoring parameters that are required for the patient.

The parameters measured by the wearable and the frequency of measurement and integration of individual measurements across time and types are not irrevocably fixed but can be changed by the wearable device and/or by a central control device which can be provided at a central server as trend and absolute data indicates an increasing risk of deterioration. In other words, while with a lower risk of deterioration the frequency of obtaining data might be lower and the set of monitoring parameters might be restricted to a sub-group, at an increasing risk of deterioration the frequency of obtaining data from the individual patient might be increased and data for the full set of monitoring parameters might be used. Some environmental feedback or contextualisation can be used to modify the implementation of the wearable device.

Preferably, the wearable device is a portable device such that remote monitoring may be performed continuously and may not restrain a patient in his daily routine. The wearable device may include at least one measurement sensor for measuring at least one of the set of monitoring parameters. For example, in the case of the monitoring parameter being a patient's heart rate, the measurement sensor may include or be a wearable electrocardiogram (ECG) device. In the case of the monitoring parameter being a patient's blood pressure, the measurement sensor may include a wearable cuff. In case the of the monitoring parameter being a patient's breathing rate, the measurement sensor may include an acoustic sensor, a pressure sensor or an acceleration sensor. Patient activity can also be measured using an acceleration sensor.

The wearable device can also provide derived parameters on the basis of raw data measured by the sensors and analyzed, processed, combined and/or reduced on the basis of predetermined rules or principles. For example, acceleration raw data measured by an acceleration sensor can be used to derive patient activity monitoring parameters as well as breathing rate monitoring parameters. Acoustic data measured by a microphone can also be used to derive breathing rate monitoring parameters and additionally breathing quality monitoring parameters.

The sensitivity and specificity of exacerbation prediction by remote monitoring with a wearable device may strongly depend on the proper technology to capture the respective monitoring parameters, the choice of appropriate data volume and monitoring frequency, reliable data transmission as well as the patient's acceptance of the wearable device. Some requirements that should be fulfilled by the at least one wearable device and/or the at least one measurement sensor are robustness and reliability, validation for being used at the patient's routine home environment, long battery lifetime or real use endurance that permits a sensible regular charging schedule (e.g. every night or every week), long maintenance intervals, minimal or no operating steps for the patient, rapid measurement of monitoring parameters, capability for continuously monitoring the parameters and implementing dynamic changes, and/or conductivity two data transmission networks, such as Bluetooth and/or Wi-Fi.

For specifying the plurality of patient groups, different possible status parameters of the patient's current health status may be taken into account. The status parameters of the patient's current health status may be a complex set of parameters obtained by physical examination, clinical history taking (including environmental and occupational) and/or questionnaires. The status parameters of the patient's current health status may include, but are not limited to, the patient's current physiological and mental health and the stage, severity and previous/likely future progression of the patient's disease. Further, the patient's disease sub-type may be considered with the patient groups. As an example, in the case of diabetes, type-1 diabetes corresponds to a different sub-type than type-2 diabetes. Taking into account these status parameters, different patient groups may be defined for which different monitoring parameters may be advisable, thereby individualizing the remote monitoring process.

To each of the patient groups, a set of monitoring parameters may be assigned. For this assignment, the respective status parameters of the patient's current health status as well as clinical history data are considered. Preferably, the number of monitoring parameters is kept as low as possible in order to prevent the remote monitoring from becoming too complex. For example, each set of monitoring parameters may comprise at most seven different monitoring parameters, preferably at most five different monitoring parameters. The different set of monitoring parameters for the different patient groups may at least partly comprise the same monitoring parameters. As an example, each of the sets of monitoring parameters may comprise the patient's blood pressure and/or heart rate as a monitoring parameter.

Furthermore, additional monitoring parameters may include parameters which can be collected only when the patient actively interacts with a certain device. Particularly, the patient may be asked to complete a questionnaire or interact with a specific measurement device such as a blood pressure cuff or a pulse oximeter. The parameters which need active patient interaction are intended to be kept to a minimum in order to ensure compliance of the patient.

According to a preferred embodiment of the method, said method comprises the following further steps:
a1) stratifying a patient into one of the patient groups or into a non-intervention group according to the patient's current health status parameters and/or the patient's clinical history data;
a2) remotely measuring the patient's monitoring parameters that corresponds to the patient's patient group by use of the respective wearable device including the respective measurements devices;
a3) analyzing a correlation of a respective value of the measured monitoring parameters to establish a probability of an acute exacerbation of the patient's health condition; and
a4) adjusting the patient groups and/or the monitoring parameters of the stratified patient group and/or the patient's stratification into the patient group according to the analysis.

Here, steps a2) to a4) are performed only for a patient stratified to patient group. That is to say, steps a2) to a4) are not performed for the non-intervention group. Preferably, the method steps a1) to a4) are performed in the indicated order. In other words, the monitoring parameters are measured after the stratification into the patient groups or the non-intervention group.

According to preferred embodiment of the method, at least some of the method steps are repeated at least once, preferably several times. In other words, the method comprises a feedback loop for testing the chosen parameters, in particular the specification of the patient groups, the monitoring parameters and/or the patient's stratification into the patient group. Preferably, all method steps are repeated at least once.

The non-intervention group may be specified in an additional step a0) that is preferably carried out before step a1). In this additional step, at least one non-intervention group is defined. A patient may be stratified into one of the non-intervention groups if, from a medical and health economical point of view, this patient is not suitable for remote monitoring at home with a chance of prevention of hospitalization once the onset of an acute exacerbation is detected early enough. For example, patients in the final stages of the disease and/or with a high comorbidities factor might not benefit from remote monitoring since the progression of the disease already has caused a level of physiologic damage and multi-morbidity which is too severe to prevent hospitalization. For these patients, early detection and intervention may either not be possible or not beneficial. Similarly, some patients demonstrate a hyper acute deterioration pattern (so-called "brittle") with little, if any, prodromal phase to be identified and for effective intervention to be instituted. On the other side, remote monitoring is not advisable for patients with a very low risk for exacerbations and/or in an early stage of the disease since the life quality of the patient is decreased by the monitoring procedure in an unnecessary manner.

If the patient is stratified into one of the patient groups, the patient is seen as being suitable for remote monitoring at home with a chance of preventing or shortening hospitalization due to acute deterioration. This stratification may initially be carried out very conservatively, i.e., patients having a reduced risk of an event may be stratified into the patient groups. Here and in the following, the term "initially" may denote an early adjustment stage of the method. During this adjustment stage, the method for providing the wearable device may be repeated several times in order to test and/or adjust feedback parameters of the method. The feedback parameters preferably include at least one of the patient groups, the monitoring parameters of the stratified patient group, and the patient's stratification into the patient group.

Preferably, the value of at least some of the measured monitoring parameters is correlated to and/or depends on the probability of an acute exacerbation. Therefore, by analyzing said correlation, the initial choice of feedback parameters may be tested. Here, for safety reasons, the remote measuring of the monitoring parameters may initially take place in a hospital. As an example, if the initial choice of the feedback parameters was adequate, the remotely measured monitoring parameters should have a direct correlation with the probability of an acute exacerbation. If there is no identifiable correlation, the feedback parameters might require an adjustment (step a4)).

Therefore, by initially making a conservative choice for the feedback parameters and analyzing this choice over time, the feedback parameters may be improved individually for each patient and/or in general for all patients. This may be seen as a self-learning feedback system for the prediction of an acute exacerbation. Starting from a rather generic or general choice for the specification of the patient groups, the assignment of the respective set of monitoring parameters and/or the stratification of the patients into the patient groups, and repeating the method steps at least a few times, may result in a highly individualized method for predicting an acute exacerbation for different types of patient groups. The findings gained from one patient by the adjustment of the feedback parameters may also be transferred to other patients, thereby continuously improving the method.

According to at least one embodiment of the method, specifying the plurality of patient groups comprises specifying a plurality of first subgroups and/or specifying a plurality of second subgroups. Preferably, for each of the first subgroups, a second subgroup is specified or vice versa. Each of the first subgroups is defined by a set of risk parameters for identifying a patient's risk for high frequency exacerbations and/or a patient's sensitivity to the effects of exacerbations. Further, each of the second subgroups is defined by a set of intervention parameters for identifying a patient's susceptibility to interventions. The at least one non-intervention group may also be specified via the specification of the plurality of first subgroups and/or the plurality of second subgroups.

The first subgroups may comprise patient's health status parameters and medical history data that have shown to be related with the frequency of exacerbation and subsequent hospitalization. Among them are existence and frequency of former exacerbations, parameters from spirometric investigations as well as disease specific scores based on questionnaires. For parameters that indicate a low risk for exacerbations and/or a low sensitivity to the effects of exacerbations, the first subgroup may be allocated to a non-intervention group since intervention should not be necessary.

The second subgroups may include indicators for identifying patients that might be susceptible to interventions, that is to say, which have a high probability to benefit from consecutive following activities such as remote monitoring. Some of these second subgroups may be allocated to a non-intervention group since remote monitoring may not be safe for patients with a health status parameter that are linked to a low susceptibility to interventions.

Preferably, the stratification of the patient into a patient group and/or a non-intervention group involves stratifying the patient into at least one of the first subgroups and/or into at least one of the second subgroups. For example, a patient may be stratified into a first subgroup that is linked to a high risk for exacerbations and into a second subgroup that is linked to a high susceptibility for interventions. Such a patient may benefit from remote monitoring. On the other hand, a patient that is stratified into a first subgroup that is linked to a high risk of exacerbations and into a second subgroup that is linked to a low susceptibility for interventions may not benefit from remote monitoring and thus will be stratified into a non-intervention group.

According to a preferred embodiment of the method, the chronic disease is COPD and the set of risk parameters includes at least one of the following of the patient's current health status parameters: number and/or frequency and/or severity of previous exacerbations; time since last exacerbation; number and/or frequency of previous hospitalizations due to exacerbations in a specified time frame; type and/or quantity and/or duration and/or severity of comorbidities; severity of COPD according to the GOLD grade; body-mass-index; BODE index; age; fitness level; smoking status and smoking behavior, in particular average packs per year; occupational exposure to substances associated with respiratory disease; eosinophil and/or neutrophil count in sputum and/or in blood; genetic factors, in particular Alpha-1-Antitrypsin deficiency (AATD); FEV₁ (forced expiratory volume in one second) and FVC (forced vital capacity) value; blood's O₂ and/or CO₂ saturation; state of the alveoli; functional residual capacity (FRC); current medication; COPD assessment test (CAT) scores; patient's emotional and mental status.

The above mentioned health status parameters have shown to give a good indication of the probability of an acute exacerbation within the next year. Furthermore, these status parameters are linked to different types of COPD that show a different progression of the disease and have different indicators for an upcoming exacerbation. The BODE index (BODE being the abbreviation of: body-mass-index, airflow obstruction, dyspnea and exercise capacity) may give a more general parameter for estimating the long-term outcomes. Similarly, the disease progression may be different for genetically induced COPD or for COPD that is caused by smoking or air pollution.

Studies indicate that for patients with a high eosinophil count, responsiveness to corticosteroid therapy is increased, thus reducing the risk for high-frequency exacerbations for these patients. Also, even though the FEV₁ value does not help to predict future exacerbation (in contrast to asthma patients, where asthma attacks are directly linked to a prior drop in the FEV₁ value), the expiratory lung capacity gives a good indication for the severity of the disease and for a long-term prognosis of its progression. The first subgroups in general do not correspond to the four GOLD stages, but the severity of the disease according to the GOLD grade may be taken into account for specifying the patient groups. The progress of the disease may also be derived from the state of the alveoli, which can be determined by measuring, for example, the lung's CO diffusion capacity. The other factors mentioned above also have shown to be related to the risk of future exacerbations.

For specifying the first subgroups, these health status parameters may be combined. As an example, one of the first subgroups may have the following elements: GOLD stage II, FEV₁/FVC below 70%, high eosinophil count in sputum and/or in blood, fitness level low, ex-smoker for three years (smoked for 20 years, two packs a day), type-2 diabetes as a comorbidity, and body-mass-index above 30. An alternative first subgroup may have the following elements: GOLD stage III, FEV₁/FVC below 50%, low eosinophil count in sputum and/or in blood, fitness level high, non-smoker, genetic markers present, and body-mass-index below 20. These different types of first subgroups may have a different risk and sensitivity to exacerbations.

According to at least one embodiment of the method, the chronic disease is COPD and the set of intervention parameters includes at least one of the following of the patient's current health status parameters: previous response to COPD medication and/or to medication for comorbidities; number and/or frequency and/or severity of previous exacerbations; number and/or frequency of previous hospitalizations due to exacerbations; type and/or quantity and/or severity of comorbidities; severity according to the GOLD grade; body-mass-index; BODE index; age; gender; fitness level; smoking status and smoking behavior, in particular average packs per year; eosinophil and/or neutrophil count in sputum and/or in blood; genetic factors, in particular Alpha-1-Antitrypsin deficiency (AATD); functional residual capacity (FRC); environmental factors, in particular housing situation; CAT scores; patient's emotional and mental status.

These health status parameters may be reliable indicators of the susceptibility of a patient to an intervention. For example, for an older person with mild dementia that lives alone in the suburbs, remote monitoring may not be advisable, whereas for the same person that lives in a residential area near a hospital, remote monitoring may be a good choice. Also, for a patient with a COPD severity according to GOLD stage IV and many comorbidities, monitoring at hospital might be the better option.

The stratification of the patient groups into the first and/or second subgroups should be performed carefully. While some of the above-mentioned status parameters, such as for example history of exacerbations, may be evident from the patient's records, other parameters, such as for example spirometry parameters or blood gas analysis, can be found historically in patient notes but current values will need to be taken using the respective analytical equipment available at Pulmonology departments of hospitals or at the clinic offices of the managing Pulmonologist/physician . Additional checks, in particular for determining the patient's stratification into one of the second subgroups, may include laboratory investigations, such as blood chemistry, as well as imaging exams and functional testing. Since the prevalence of comorbidities is a major reason for complications for COPD patients, the existence and severity of comorbidities can be seen as an essential criterion for the stratification. The differential diagnosis of these comorbidities may require extensive investigations, ranging from physical examinations, like e.g. heart rate, blood pressure, ECG, laboratory examinations, such as blood analysis and immunoassay, imaging procedures as well as recording clinical history data via verbal anamnesis. Further, standardized questionnaires can be filled in paper format and/or in electronic format, for example on a smartphone or a tablet computer, and transferred into the patient's data record afterwards, either manually or automatically. Based on their history and the status of the respective health parameters, patients may be stratified into at least one up to a number of first and/or second subgroups.

According to at least one embodiment of the method, the chronic disease is COPD and the set of monitoring parameters includes at least one of the following patient's health parameters: arterial blood gas, in particular partial pressure of CO₂ and O₂; inflammation score such as ESR, CRP or more specialized autoantibody titers; fraction of exhaled NO and/or *NOₓ (x ≥ 2) as inflammation marker; respiratory rate; long-term respiratory supplemental sounds; eosinophil and/or neutrophil count in sputum and/or in blood; macrophage count in sputum; inspiratory flow rate; CO diffusion capacity; heart rate; heart rate variability; cardiac rhythm disturbances; blood pressure; physical activity; fitness level; quality of sleep; frequency of cough; amount and color of mucus; CAT scores; BODE index; patient's emotional and mental state.

In particular, the set of monitoring parameters may comprise a static set of parameters which may be common for all patient groups and/or a dynamic set of parameters that is specific for the respective patient group. Further, physiologic performance data for each patient may be added or substituted depending on the patient's anamnesis and/or after a period of monitoring.

Suitable measurement sensors for capturing the above-mentioned parameters may include classic spirometers or pulse oximeters, but also modern multi-parameter wearable devices automatically and continuously recording the respective physiologic parameters without the need of the patient's action. Such wearable devices may have the capability to remotely transfer data to databases that are used for analysis of the measured monitoring parameters. For some of the parameters, continuous monitoring may be preferred, for example in the case of an oxygen saturation that is measured with a pulse oximeter, a drop of saturation under exercise is an earlier and more powerful indicator of physiological decompensation and potential deterioration than a one-time early-morning measurement under rest.

According to at least one embodiment of the method, at least one of the monitoring parameters is remotely measured by use of the patient's feedback. Preferably, the patient's feedback is obtained via a structured but contextually specific set of questions. These may validated clinical questionnaires or customized ones developed specifically for remote monitoring. Similarly they may utilize fixed questions or adapts the interrogatory approach depending on previous responses. Such a questionnaire may, for example, be performed on a regular basis by the use of an electronic device, for example a smartphone or tablet computer. With such a questionnaire, individual parameters reflecting the health status of the patient may be obtained. For example, CAT scores or the BODE index may be calculated from such questionnaires. In addition, a change in the patient's emotional health may be detected early. In particular, depression may have a major impact on the probability of an exacerbation.

According to at least one embodiment of the method, selecting at least one wearable device includes the step of providing at least one universal wearable device, comprising a plurality of measurement sensors, the universal wearable device being adapted for measuring the respective monitoring parameters of at least two of the patient groups. The method further involves the step of programming the universal wearable device such that the measurement sensors for the respective monitoring parameters of one of the at least two patient groups are monitored. One advantage of programming the wearable device such that only the required monitoring parameters are measured is savings in battery capacity, thereby making the wearable device more long-lived.

According to at least one embodiment of the method, selecting the at least one wearable device includes the step of, for each of the monitoring parameters of all patient groups, selecting at least one measurement sensor for remotely measuring said monitoring parameter. In a further step, the wearable device is provided by combining the respective measurement sensors for the monitoring parameters for each of the patient groups. That is to say, the constituents of the respective wearable device may be arranged individually for each of the patient groups.

Further, a method for predicting an acute exacerbation of a health condition of a patient with a chronic disease, in particular a COPD patient, is provided. The method is preferably carried out with a wearable device that is provided with a method described herein. That is to say, all features that are disclosed in connection with the method for providing a wearable device are also disclosed for the method for predicting an acute exacerbation and vice versa.

Accordingly, the method for predicting an acute exacerbation of a health condition of a patient with a chronic disease, in particular a COPD patient, comprises the following steps:
b1) specifying a plurality of patient groups, wherein each of the patient groups is defined by a set of status parameters of the patient's current health status and/or the patient's medical history data;
b2) assigning each of the patient groups to a respective set of monitoring parameters of a patient;
b3) stratifying the patient into one of the patient groups or into a non-intervention group according to the patient's current health status and/or the patient's clinical history data;
b4) remotely measuring the monitoring parameters of the patient that correspond to the patient's patient group;
b5) determining a theoretical probability of an acute exacerbation of the patient's condition by analyzing each of the measured monitoring parameters according to a pre-defined scheme;
b6) generating a warning signal if the theoretical probability of an acute exacerbation exceeds a predetermined value.

Here, steps b4) to b6) are performed only for a patient that had been stratified to a patient group. In other words, steps b4) to b6) are not performed for the non-intervention group. Preferably, at least some of the method steps b1) to b4) are performed in the indicated order.

Determining a theoretical probability of an acute exacerbation by analyzing measured monitoring parameters (step b5)) may involve mathematical models and algorithms for the progression of the chronic disease. With such mathematical models or algorithms, the actual patient specific data can be compared with a control group of the same or similar patient group, with an age-matched control group, with a baseline that is specific for the patient group, and/or even with a patient-specific baseline. The patient-specific baseline may, for example, be determined from long-term measurement data. Intra-patient stability of exacerbation symptoms appear to be relatively stable and might act as a very suitable baseline for detecting early deteriorations. Any kind of bio-statistical modeling or algorithm can be suitable for conducting this comparison. However, preferably machine learning technologies may be exploited for implementing such an algorithm.

One of the purposes of analyzing the remotely monitored parameters, in particular by the use of data comparison, is to detect deviations from predetermined values, such as predetermined specifications and/or limits, and/or trends which indicate an upcoming acute worsening of the patient's condition. The database and algorithms may be designed in a way that the algorithms can be refined during data collection by use of the remote monitoring, thus leading to improvements of predictive accuracy, specificity and selectivity.

The determination of the theoretical probability of an acute exacerbation may be carried out in a dedicated intelligent database system (clinical decision systems, CDS). In these databases, the initially measured and obtained patient data as well as the remotely monitored parameters of the patient may be collected, stored and compared with each other. These databases should be compliant with all requirements for data securities, safety and discretion stipulated in the relevant territories. The corresponding data center for the databases may be supervised by healthcare professionals and/or a healthcare provider.

Once a deviation from a predetermined specification and/or limit is detected and the trend analysis points at the possibility of an upcoming severe exacerbation, a warning signal is raised. The warning signal may, for example, be an optical signal on a screen, which may be continuously and/or regularly supervised by a healthcare professional. It is also possible for the CDS to trigger a feedback to the patient and ask him to contact his healthcare provider and/or to initiate pre-agreed therapeutic interventions. The CDS may also call the patient.

According to at least one embodiment of the methods, analyzing of the measured monitoring parameters (step b5)) includes at least one of the following processes or a combination of at least two of the following processes: weighting the measured monitoring parameter over time; comparing the measured monitoring parameter and/or weighted measured monitoring parameter with a predefined threshold value; determining a periodicity of the measured monitoring parameter; determining a change rate of the measured monitoring parameter. As an example, by weighting the measured monitoring parameters over time, an individual baseline level for each patient may be determined. A change in the monitoring parameters, for example in its periodicity, may indicate an upcoming worsening of the patient's health status.

According to at least one embodiment of the method, the remote monitoring is carried out continuously. That is to say, the monitoring is carried out on a regular basis and preferably periodically. A sampling frequency for a periodic measurement may be established individually for each patient group and/or for each patient. An increase in risk of deterioration, which can be inferred from the trend of the monitoring parameters and/or from environmental or medicinal changes, may also trigger a change of the sampling frequency. The method may thus involve a dynamic and adaptive design.

According to at least one embodiment, the method further comprises the step of determining the real probability of an acute exacerbation. This real probability is then compared with the determined theoretical probability (step b5) mentioned above). The method comprises a further step of adjusting the patient groups, the monitoring parameters of the stratified patient group and/or the patient's stratification into the patient group according to the comparison. The method may thus involve a feedback for improving the chosen parameters, in particular the feedback parameters mentioned above in connection with the method for providing the wearable device.

Furthermore, a system for predicting an acute exacerbation of a health condition of a patient with a chronic disease, in particular a COPD patient, is provided. The system is preferably adapted for performing a method for predicting an acute exacerbation described herein. Further, the system preferably comprises a wearable device that is provided by a method as described herein. That is to say, all features that are disclosed in connection with the method for predicting an acute exacerbation and/or in connection with the method for providing a wearable device are also disclosed for the system for predicting an acute exacerbation and vice versa.

The system comprises a monitoring server, a wearable device and a warning signal generator. The monitoring server is adapted for specifying a plurality of patient groups, assigning each of the patient groups to a respective set of monitoring parameters of a patient, stratifying the patient into one of the patient groups or into a non-intervention group, and determining a theoretical probability of an acute exacerbation of the patient's condition by analyzing measured monitoring parameters for at least some of the patient groups according to a predefined scheme. The wearable device is adapted for remotely measuring the monitoring parameters of the patient that correspond to the patient's patient group. Further, the warning signal generator is adapted for generating a warning signal if the theoretical probability of an acute exacerbation exceeds a predetermined value.

The monitoring server may be a clinical decision system (CDS) in connection with a healthcare provider. For example, the monitoring server may comprise a database for storing and/or collecting data. The data that might be stored in the monitoring server may comprise historic patient data taken from former hospitalizations (e.g., biochemical, microbiological, genetic information or information from imaging technologies). This historic data may be used for stratifying the patient into a patient group. Further, the data that is produced by the remote monitoring may be stored in the monitoring server and compared with predefined threshold values in order to determine a probability for an acute exacerbation. Data transfer from and/or to the monitoring server may be carried out via modem, LAN, WLAN, Bluetooth, GSM, LTE, cloud based systems and/or Wi-Fi or any other wired or wireless communication system

According to at least one embodiment of the system, the wearable device comprises at least one of the following components: a GPS component that is adapted for determining the patient's position, a transmission component for exchanging data between the wearable device, the warning signal generator and/or the monitoring server, an interface for exchanging data between the wearable device and a further device, in particular a mobile device, such as a smartphone and/or a tablet computer.

These additional components may be helpful for estimating a patient's risk of an acute exacerbation. For example, GPS data can be helpful for patient movement surveillance, either to determine the patient's environment (as for example height or humidity) or to locate a patient in case of danger. Activity trackers can assist in determining the level of physical activity, which may also be helpful for deriving the patient's mental health status. Data from meteorological services may be received with a mobile device and transmitted to the wearable device and/or the monitoring server. These data may provide important supportive information. For example, some studies suggest a potential relation between actual meteorological and/or environmental conditions, such as temperature, humidity, pollution, and worsening of the patient's conditions.

### Brief description of the drawings

Preferred embodiments and detailed examples of the invention will be explained in the following, having regard to the drawings.
Figure 1 schematically shows an embodiment of a method and a system for predicting an acute exacerbation.
Figure 2 schematically shows an embodiment of a method for providing a wearable device, a method and a system for predicting an acute exacerbation.
Figure 3 schematically shows an embodiment of a method for providing a wearable device and a method for predicting an acute exacerbation.

### Detailed description of preferred embodiments

In the following, preferred embodiments of the invention will be described with reference to the drawings. Here, elements that are identical, similar or have an identical or similar effect are provided with the same reference numerals in the figures. The figures and the size relationships of the elements illustrated in the figures among one another should not be regarded as to scale. Rather, individual elements may be illustrated with an exaggerated size to enable better illustration and/or better understanding.

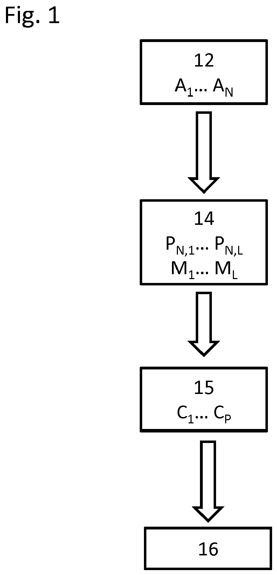

With reference to the schematic diagram of Figure 1, an embodiment of a method for providing a wearable device and for predicting an acute exacerbation according to the present invention is described in detail.

The system comprises a monitoring server 12, 15, a wearable device 14 and a warning signal generator 16. The monitoring server comprises a stratification server 12 for stratifying the patient into patient groups A₁,...,A_{N} (N being an index with N>1 In the corresponding method step, the patient is stratified into one of the patient groups A₁,... ,A_{N} or into a non-intervention group N₁,...,N_{Q} (Q being an index with Q>1 ). If the patient is stratified into a patient group AN, the monitoring parameters P_{N,1},...,P_{N,L} (L being an index with L>1) that correspond to the stratified patient group AN are remotely monitored with a plurality of measurement sensors M₁,...,M_{L} of the wearable device 14.

The measured monitoring parameters P_{N,1},...,P_{N,L} are then evaluated by use of an evaluation server 15 of the monitoring server 12, 15, wherein the monitored parameters P_{N,1},...,P_{N,L} may be compared to predefined threshold values C₁,..., C_{P} (P being an index with P>1. Depending on the output of the evaluation server 15, the warning signal generator 16 may generate a warning signal and/or trigger an intervention.

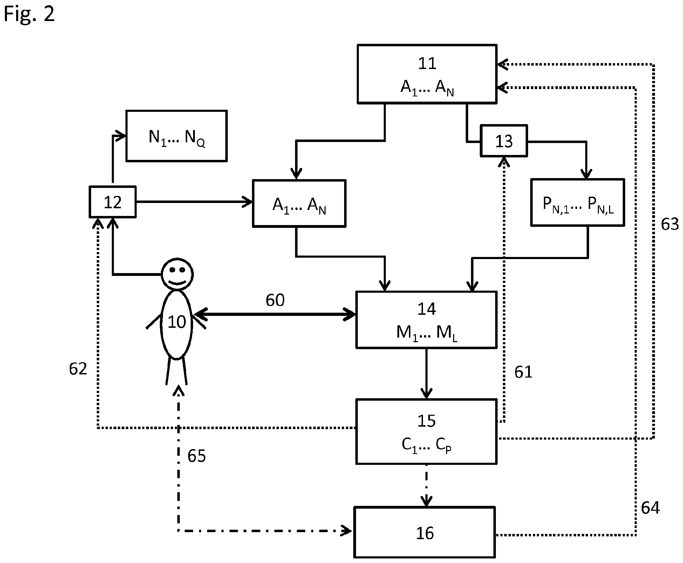

With reference to the schematic diagram of Figure 2, an embodiment of a method for providing a wearable device 14, an embodiment of a method for predicting an acute exacerbation and an embodiment of a system for predicting an acute exacerbation according to the present invention are explained in detail.

In an initial method step, the patient groups A₁,...,A_{N} are specified by use of a specification server 11 that is part of the monitoring server. A set of monitoring parameters P_{N,1},...,P_{N,L} is assigned to each of the patient groups by use of an assignment server 13, which is also part of the monitoring server.

For each of the patient groups A₁,...,A_{N}, at least one wearable device 14 may be selected according to the set of monitoring parameters P_{N,1},...,P_{N,L} of the patient group A₁,...,A_{N}. The wearable device 14 may comprise at least one measurement sensor for monitoring at least one of the monitoring parameters of the set of monitoring parameters P_{N,1},...,P_{N,L}.

The stratification server 12 is used to stratify a patient 10 into one of the patient groups A₁,...,A_{N} or into a non-intervention group N₁,...,N_{Q}. If the patient 10 has been stratified into a non-intervention group N₁,...,N_{Q}, no further monitoring measures may be taken. However, if the patient 10 has been stratified into one of the patient groups A₁,... ,A_{N}, the set of monitoring parameters P_{N,1},...P_{N,L} of this patient A₁,...,A_{N} group is remotely measured 60 by use of the wearable device 14.

From the measured monitoring parameters P_{N,1},...,P_{N,L}, a theoretical probability of an acute exacerbation is determined with the evaluation server 15 by comparing the measured monitoring parameters P_{N,1},...,P_{N,L}, with predefined threshold values C₁,..., C_{P} and/or by exploiting mathematical algorithms and/or statistical models. Depending on the determined theoretical probability, the warning signal generator 16 may generate a warning signal that is transmitted to the patient via a patient feedback 65.

The results of the evaluation server 15 may be fed back to the assignment server 13 via a first feedback loop 61, to the stratification server 12 via a second feedback loop 62, and/or to the specification server 11 via a third feedback loop 63. Owing to this feedback, the specification of the patient groups A₁,...A_{N,} the assignment of the monitoring parameters P_{N,1},...,P_{N,L} of the stratified patient group A₁,...,A_{N} and/or the patient's stratification into the patient group A₁,...,A_{N} can be adjusted. Further, the results the warning signal generator 16 may be fed back to the specification server 11 via a fourth feedback loop 64. With this fourth feedback loop 64, further adjustment of the specification of the patient groups A₁,...A_{N} is possible, in particular in the case of frequent warnings.

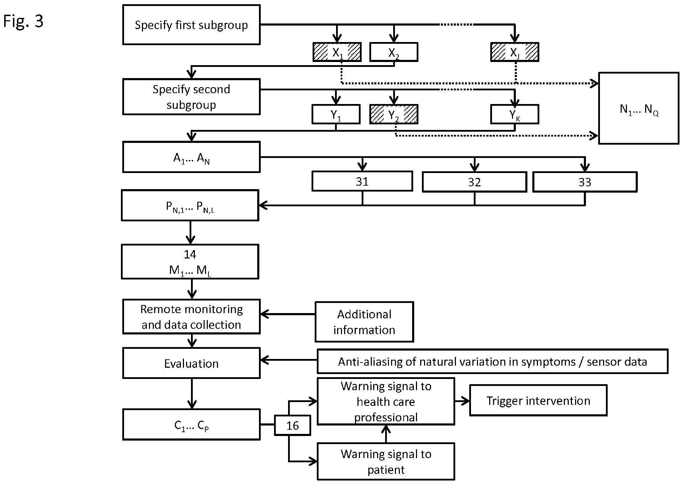

With reference to the schematic diagram of Figure 3, an embodiment of a method for providing a wearable device 14 and an embodiment of a method for predicting an acute exacerbation according to the present invention are explained in detail.

In the method, first subgroups X₁,...,X_{J} and second subgroups Y₁,...,Y_{K} are specified. Each of the first subgroups X₁,...,X_{J} may be defined by a set of risk parameters for identifying a patient's risk for high frequency exacerbations and/or a patient's sensitivity to the effects of exacerbations. At least one of the first subgroups X₁, X_{J} (indicated shaded in Figure 3) may correspond to a low probability of exacerbations and/or to a low sensitivity to exacerbations. For example, these first subgroups with a low risk parameter X₁, X_{J} may correspond to an early COPD stage according to the GOLD grade.

These first subgroups with a low risk parameter X₁, X_{J} can be assigned to a non-intervention group N₁,...,N_{Q}.

Each of the second subgroups Y₁,...,Y_{K} may be defined by a set of intervention parameters for identifying a patient's susceptibility to interventions. At least one of the second subgroups Y₂ (indicated shaded in Figure 3) may correspond to a low susceptibility to interventions and can thus be assigned to a non-intervention group N₁,...,N_{Q}.

The first subgroups X₁,...,X_{J} and second subgroups Y₁,...,Y_{K} that are not assigned to a non-intervention group N₁,...,N_{Q} may constitute the patient groups A₁,...A_{N}. For these patient groups, respective sets of monitoring parameters P_{N,1},...,P_{N,L} may be defined by taking into account general parameters 31, custom parameters 32 and individual parameters 33. General parameters 31 are identical for all patient groups A₁,...A_{N,} Custom parameters 32 may only be monitored in some of the patient groups A₁,...A_{N,} And individual parameters 33 may individually be assigned to a patient.

According to the specific set of monitoring parameters P_{N,1},...,P_{N,L} a wearable device 14 is selected, for example by programming a universal wearable device, and the patient is remotely monitored by use of the wearable device 14. Here, additional information may be taken into account for adjusting the wearable device 14 and/or subsequent data analysis. The additional information may for example comprise monitoring and analysis settings, such as changes to the sampling frequency or sampling thresholds, as well as parameter weighting in response to the data. Furthermore, environmental and/or historic patient data may be provided to the wearable device 14. Therefore, it is possible to adjust the measurement depending on the conditions of the patient.

The collected data is evaluated by use of, for instance, mathematical algorithms. Here, anti-aliasing may be applied by considering natural variations in the measured symptoms and/or the sensor data. For this, an individual background level of the patient can be taken into account, thereby adjusting the method to the patient.

The evaluation results in a theoretical probability of an acute exacerbation. This determined theoretical probability may be compared to a predetermined threshold value C₁,..., C_{P}. In addition or as an alternative, it is possible to compare at least one of the measured monitoring parameters P_{N},₁,...,P_{N,L} with a predetermined threshold value C₁,..., C_{P}. If the theoretical probability (and/or the measured monitoring parameters P_{N,1},...,P_{N,L}) exceeds the threshold value C₁,..., C_{P}, a warning signal may be generated by the warning signal generator 16 and sent to the patient and/or the responsible health care professional. The health care professional may then decide whether an intervention, such as a change of medication and/or at hospital treatment, might be advisable.

In the following, a first example for specifying the patient groups A₁,...A_{N} and stratifying a patient according to a method for providing a wearable device 14 as well as for the method for predicting an acute exacerbation according to the present invention will be described in detail. In this first example, two patient groups A₁, A₂ (i.e. N=2) are chosen for simplicity.

In a first step of the method, a plurality of first subgroups X₁,...,X_{J} and a plurality of second subgroups Y₁,...,Y_{K} are defined. As a mere example, three first subgroups X₁, X₂, X₃ (i.e. J=3) and three second subgroups Y₁, Y₂, Y₃ (i.e. K=2) are chosen. The risk parameters of the "first" first subgroup X₁ correspond to a patient having had no acute exacerbation within the last year, an FEV₁/FVC value above 70% and a GOLD stage of I. The risk parameters of the "second" first subgroup X₂ correspond to a patient having had at least one acute exacerbation within the last year, an FEV₁/FVC value above 40% and below 70% and/or a GOLD stage of II. The risk parameters of the "third" first subgroup X₃ correspond to a patient having had more than one acute exacerbation within the last year, an FEV₁/FVC value below 40% and/or a GOLD stage of III or IV. The "first" first subgroup X₁ is assigned to a first non-intervention group N₁ since the risk for an exacerbation is very low. Further, the "third" first subgroup X₃ is assigned to a second non-intervention group N₂ since the chance for high-frequency exacerbations is too high and/or the patient may not benefit from remote monitoring.

The intervention parameters of the "first" second subgroup Y₁ may correspond to a patient having at most one comorbidity, an age below 50 and is living with a spouse or family. The intervention parameters of the "second" second subgroup Y₂ may correspond to a patient having at most one comorbidity, an age below 70 and is living alone. The intervention parameters of the "third" second subgroup Y₃ may correspond to a patient having more than two comorbidities, an age above 70 and/or is living alone. The "third" second subgroup Y₃ is assigned to a third non-intervention group N₃ since the patient may not benefit from remote monitoring. Patients that do not fulfill any of the requirements for any of the first subgroups X₁, X₂, X₃ or any of the second subgroups Y₁, Y₂ are stratified into a fourth non-intervention group N₄.

The first patient group A₁ may then be specified as the combination of the "second" first subgroup X₁ and the "first" second subgroup Y₁, i.e. A₁=( X₂, Y₁). Similarly, the second patient group A₂ may be specified as the combination of the "second" first subgroup X₁ and the "second" second subgroup Y₂, i.e. A₁=(X₂, Y₂). For both patient groups A₁, A₂, the respective set of monitoring parameters P_{N,1},...,P_{N,L} may include heart rate (P₁₁ and P₂₁), heart rate variability (P₁₂ and P₂₂), arterial oxygen saturation (P₁₃ and P₂₃), inspiratory flow rate (P₁₄ and P₂₄), and blood pressure (P₁₅ and P₂₅). However, in the case of the second patient group A₂, increased monitoring might be useful due to the higher age of the patient or missing feedback by a roommate. The set of monitoring parameters may then further include long-term respiratory noise (P₂₅), frequency of cough (P₂₆), and amount and color of mucus (P₂₇).

The invention is not restricted by the description based on the embodiments and examples. Rather, the invention comprises any new feature and also any combination of features, including in particular any combination of features in the patent claims, even if this feature or this combination itself is not explicitly specified in the patent claims or exemplary embodiments.

### List of reference numerals

- 11: Specification server
- 12: Stratification server
- 13: Assignment server
- 14: Wearable device
- 15: Evaluation server
- 16: Warning signal generator
- 31: General parameters
- 32: Custom parameters
- 33: Individual parameters
- 60: Remote measurement
- 61: First feedback loop
- 62: Second feedback loop
- 63: Third feedback loop
- 64: Fourth feedback loop
- 65: Patient feedback
- A₁,...A_{N}: Patient groups
- P_{N,1},...,P_{N,L}: Set of monitoring parameters
- N₁,...,N_{Q}: Non-intervention groups
- M₁,...,M_{L}: Measurement sensors
- C₁,..., C_{P}: Threshold values
- X₁,...,X_{J}: First subgroups
- Y₁,...,Y_{K}: Second subgroups

## Claims

1. Method for providing a wearable device (14) for remotely monitoring a patient with a chronic disease, in particular a COPD patient, the method comprising the steps of:
- specifying a plurality of patient groups (A₁,...,A_{N}), wherein each of the patient groups (A₁,...A_{N}) is defined by at least a set of status parameters of the patient's current health status and/or the patient's clinical history data;
- assigning each of the patient groups (A₁,...,A_{N}) to a respective set of monitoring parameters (P_{N,1},..,P_{N,L});
- for each of the patient groups (A₁,...,A_{N}), selecting at least one wearable device (14) that includes measurement sensors for remotely measuring at least one of the monitoring parameters (P_{N,1},..., P_{N,L}) of the respective set of monitoring parameters (P_{N,1},..., P_{N,L}) of the patient group (A₁,...,A_{N}).

2. Method according to the previous claim, further comprising the following steps:
a1) stratifying a patient into one of the patient groups (A₁,...A_{N}) or into a non-intervention group (N₁,...,N_{Q}) according to the patient's current health status parameters and/or the patient's clinical history data,
a2) remotely measuring the patient's monitoring parameters (P_{N,1},...,P_{N,L}) that correspond to the patient's patient group (A₁,...,A_{N}) by use of the respective wearable device including the respective measurement sensors (M₁,...,M_{L});
a3) analyzing a correlation of a respective value of the measured monitoring parameters (P_{N,1},...,P_{N,L}) from a probability of an acute exacerbation of the patient's health condition; and
a4) adjusting the patient groups (A₁,...A_{N}) and/or the monitoring parameters (P_{N,1},..., P_{N,L}) of the stratified patient group (A₁,...,A_{N}) and/or the patient's stratification into the patient group (A₁,...,A_{N}) according to the analysis,
wherein steps a2) to a4) are performed only for a patient stratified into a patient group (A₁,...A_{N}).

3. Method according to the previous claim, wherein at least some of the method steps are repeated at least once.

4. Method according to any of the preceding claims, wherein specifying the plurality of patient groups (A₁,...,A_{N}) comprises at least one of the following steps:
- specifying a plurality of first subgroups (X₁,... ,X_{J}), wherein each of the first subgroups (X₁,...,X_{J}) is defined by a set of risk parameters for identifying a patient's risk for high frequency exacerbations and/or a patient's sensitivity to the effects of exacerbations;
- specifying a plurality of second subgroups (Y₁,...,Y_{K}), wherein each of the second subgroups (Y_{J,1},...,Y_{J,K}) is defined by a the set of intervention parameters for identifying a patient's susceptibility to interventions.

5. Method according to the previous claim, wherein the chronic disease is COPD and the set of risk parameters includes at least one of the following of the patient's current health status parameters:
number and/or frequency and/or severity of previous exacerbations; time since last exacerbation; number and/or frequency of previous hospitalizations due to exacerbations;
type and/or quantity and/or severity of comorbidities; severity according to the GOLD grade;
body-mass-index; BODE index; age; fitness level; smoking status and smoking behavior, in particular average packs per year; eosinophil and/or neutrophil count in sputum and/or in blood; genetic factors, in particular Alpha-1-Antitrypsin deficiency (AATD); FEV₁ (forced expiratory volume in one second) and FVC (forced vital capacity) value; blood's O₂ and/or CO₂ saturation; state of the alveoli; functional residual capacity (FRC); current medication;
COPD assessment test (CAT) scores; patient's emotional and mental status.

6. Method according to any of the two previous claims, wherein the chronic disease is COPD and the set of intervention parameters includes at least one of the following of the patient's current health status parameters:
previous response to COPD medication and/or to medication for comorbidities; number and/or frequency and/or severity of previous exacerbations; number and/or frequency of previous hospitalizations due to exacerbations; type and/or quantity and/or severity of comorbidities;
severity according to the GOLD grade; body-mass-index; BODE index; age; gender; fitness level; smoking status and smoking behavior, in particular average packs per year; eosinophil and/or neutrophil count in sputum and/or in blood; genetic factors, in particular Alpha-1-Antitrypsin deficiency (AATD); functional residual capacity (FRC); environmental factors, in particular housing situation; CAT scores; patient's emotional and mental status.

7. Method according to any of the previous claims, wherein the chronic disease is COPD and the set of monitoring parameters (P_{N,1},...,P_{N,L}) includes at least one of the following patient's health parameters:
arterial blood gas, in particular saturation of CO₂ and O₂; inflammation score; fraction of exhaled NO and/or NOₓ (x ≥ 2) as inflammation marker; long-term respiratory noise;
respiratory rate, eosinophil and/or neutrophil count in sputum and/or in blood; macrophage count in sputum; inspiratory flow rate; CO diffusion capacity; heart rate; heart rate variability;
blood pressure; physical activity; fitness level; quality of sleep; frequency of cough; amount and color of mucus; CAT scores; patient's emotional and mental status.

8. Method according to any of the previous claims, wherein at least one of the monitoring parameters (P_{N,1},... ,P_{N,L)} is remotely measured by use of the patient's feedback, in particular via a questionnaire.

9. Method according to any of the previous claims, wherein selecting the at least one wearable device (14) includes the steps of:
- providing at least one universal wearable device, comprising a plurality of measurement sensors, that is adapted for measuring the respective monitoring parameters (P_{N,1},... ,P_{N,L}) of at least two of the patient groups (A₁,...,A_{N});
- programming the universal wearable device such that the measurement sensors for the respective monitoring parameters (P_{N,1},...,P_{N,L}) of one of the at least two patient groups (A₁,...,A_{N}) are monitored.

10. Method according to any of the previous claims, wherein selecting the at least one wearable device (14) includes the steps of:
- for each of the monitoring parameters (P_{N,1},..., P_{N,L}) of all patient groups (A₁,... ,A_{N}), selecting at least one measurement sensor (M₁,...,M_{L} for remotely measuring said monitoring parameter (P_{N,1},...,P_{N,L});
- providing the wearable device (14) by combining the respective measurement sensors (M₁,..., M_{L}) for the monitoring parameters (P_{N,1},...,P_{N,L}) for each of the patient groups (A₁,...,A_{N}).

11. Method for predicting an acute exacerbation of a health condition of a patient with a chronic disease, in particular a COPD patient, the method comprising:
b1) specifying a plurality of patient groups (A₁,...,A_{N}), wherein each of the patient groups (A₁,...A_{N}) is defined by a set of status parameters of the patient's current health status and/or the patient's clinical history data;
b2) assigning each of the patient groups (A₁,...,A_{N}) to a respective set of monitoring parameters (P_{N,1},..., P_{N,L}) of a patient;
b3) stratifying the patient into one of the patient groups (A₁,...,A_{N}) or into a non-intervention group (N₁,,...,N_{Q}) according to the patient's current health status parameters and/or the patient's clinical history data;
b4) remotely measuring the monitoring parameters (P_{N,1},...,P_{N,L}) of the patient that correspond to the patient's patient group (A₁,...,A_{N});
b5) determining a theoretical probability of an acute exacerbation of the patient's condition by analyzing each of the measured monitoring parameters (P_{N,1},..., P_{N,L}) according to a pre-defined scheme;
b6) generating a warning signal if the theoretical probability of an acute exacerbation exceeds a predetermined value,
wherein steps b4) to b6) are performed only for a patient stratified to a patient group (A₁,...A_{N}).

12. Method according to the previous claim, wherein analyzing of a measured monitoring parameter (P_{N,1,}...P_{N,L}) includes at least one of the following processes or a combination of at least two of the following processes:
- weighting the measured monitoring parameter (P_{N,1,}...P_{N,L}) over time;
- comparing the measured monitoring parameter (P_{N,1,}...P_{N,L}) and/or the weighted measured monitoring parameter with a predefined threshold value (C₁,..., C_{P});
- determining a periodicity of the measured monitoring parameter (P_{N,1},..., P_{N,L});
- determining a change rate of the measured monitoring parameter (P_{N,1},..., P_{N,L}).

13. Method according to any of the two previous claims, wherein the remote monitoring is carried out continuously.

14. Method according to any of claims 10 to 13, further comprising the following steps:
- determining the real probability of an acute exacerbation;
- comparing the real probability with the theoretical probability;
- adjusting the patient groups (A₁,...A_{N}) and/or the monitoring parameters (M₁,..., M_{L}) of the stratified patient group (A₁,...A_{N}) and/or the patient's stratification into the patient group (A₁,...A_{N}) according to the comparison.

15. System for predicting an acute exacerbation of a health condition of a patient with a chronic disease, in particular a COPD patient, comprising
- a monitoring server (11, 12, 13, 15) that is adapted for specifying a plurality of patient groups (A₁,...,A_{N}), assigning each of the patient groups (A₁,...,A_{N}) to a respective set of monitoring parameters (P_{N,1},...,P_{N,L}) of a patient, stratifying the patient into one of the patient groups (A₁,...,A_{N}) or into a non-intervention group (N₁,...,N_{Q}), and determining a theoretical probability of an acute exacerbation of the patient's condition by analyzing measured monitoring parameters (P_{N,1},...,P_{N,L}) for at least some of the patient groups (A₁,...,A_{N}) according to a predefined scheme,
- a wearable device (14) that is adapted for remotely measuring the monitoring parameters (P_{N,1},..., P_{N,L}) of the patient that correspond to the patient's patient group (A₁,...A_{N}), and
- a warning signal generator (16) that is adapted generating a warning signal if the theoretical probability of an acute exacerbation exceeds a predetermined value

16. System according to the previous claim, wherein the wearable device comprises at least one of the following components:
- a GPS component that is adapted for determining the patient's position,
- an activity tracker for determining the patient's level of physical activity,
- a transmission component for exchanging data in between the wearable device, the warning signal generator and/or the monitoring server,
- an interface for exchanging data in between the wearable device and a further device, in particular a mobile device.
